(19) Europäisches Patentamt
European Patent Office
Office européen des brevets

(11) Publication number: 0 313 235 A2

# (12) EUROPEAN PATENT APPLICATION

(21) Application number: 88309328.8

(51) Int. Cl.4: C09K 19/20 , G02F 1/13

(22) Date of filing: 06.10.88

(30) Priority: 22.10.87 GB 8724762

(43) Date of publication of application: 26.04.89 Bulletin 89/17

(84) Designated Contracting States: AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Colquhoun, Howard Matthew
8 Grove Park
Knutsford Cheshire WA16 8QA(GB)
Inventor: Dudman, Christopher Curtis
9 Edwards Road
Lache Park§Chester CH4 8HW(GB)
Inventor: Gemmell, Peter Alan
4 Silver Leys
Bentley Ipswich Suffolk(GB)
Inventor: Robinson, Gareth Charles
41 Hatherton Way
Chester Cheshire CH2 2AC(GB)

(74) Representative: Collingwood, Anthony Robert et al
Imperial Chemical Industries PLC Legal Department: Patents P.O. Box 6 Bessemer Road
Welwyn Garden City Hertfordshire AL7 1HD(GB)

## (54) Liquid crystal device.

(57) A liquid crystal device in which the liquid crystal material comprises or contains a compound having ferro-electric spontaneous polarisation properties, the compound being of the formula:

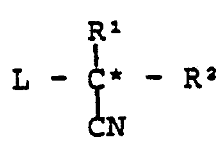

Where L is a liquid crystal residue or liquid crystal compatible material having a terminal cyclic structure directly connected to the asymmetric carbon centre C*, $R^1$ is selected from $C_{1-4}$-alkyl and halogen and $R^2$ is selected from alkyl, alkoxy, alkylthio, halo-alkyl & -alkoxy, alkoxyalkyl, halogeno, alkyl-carbonyl & -carbonyloxy and alkoxy -carbonyl and -carbonyloxy.

EP 0 313 235 A2

# LIQUID CRYSTAL DEVICE

This invention relates to liquid crystal devices, such as display or data storage devices, and to a novel compound having ferro-electric properties and its use in liquid crystal devices.

According to one aspect of the present invention there is provided a liquid crystal device comprising means enclosing a liquid crystal material and means for subjecting the liquid crystal material to an electric or magnetic field, the liquid crystal material comprising or containing a compound which has spontaneous polarisation properties and is of of the formula:

$$\begin{array}{c} \quad\quad\quad\;\; R^1 \\ \quad\quad\quad\;\; | \\ A-T-A^1-C^*-R^2 \\ \quad\quad\quad\;\; | \\ \quad\quad\quad\;\; CN \end{array} \qquad\qquad I$$

wherein :

A-T-$A^1$ is the residue of a liquid crystal material or a liquid crystal compatible material, having a terminal cyclic structure, $A^1$, connected to the asymmetric carbon atom, $C^*$, by a single covalent bond;

$A^1$ is selected from: optionally substituted 1,4-phenylene optionally with one to four cyclic carbon atoms replaced by N; optionally substituted 1,4-cyclohexylene optionally with one or two cyclic carbon atoms replaced by O, S or N; 1,4-bicyclo (2,2,2) octane; 2,6-naphthylene or 1,4-naphthylene;

T is selected from a single covalent bond, or a group selected from -CO.O-, -O.CO, -CO.S-, CS.O-, $-CH=CH-$, $-CH_2O-$, $-OCH_2-$, $-N=\underset{\substack{\downarrow\\ O}}{N}-$, $-N=N-$, $-\underset{\substack{\downarrow\\ O}}{N}=N-$, $-N=CH-$, $-CH=N-$, $-CH_2CHE-$, $-CHECH_2-$, and $-C\equiv C-$;

E is selected from H, cyano, halogen, $CF_3$ and $CH_3$;

$R^1$ is selected from $C_{1-4}$-alkyl and halogen; $R^2$ is selected from alkyl, alkoxy, alkylthio, halo-alkyl & -alkoxy, alkoxyalkyl, halogeno, alkyl-carbonyl & -carbonyloxy and alkoxy-carbonyl and -carbonyloxy;

A is the remainder of the liquid crystal residue or liquid crystal compatible material;

provided that $R^1$ and $R^2$ are different.

Also, according to the invention there is provided a compound as defined above and the use of such a compound as a liquid crystal material or in combination with a liquid crystal material in a liquid crystal device such as a display device or a data storage device.

The residue of the liquid crystal, or liquid crystal compatible, material is preferably of the formula:

$$R^3-(A^4)_k-T^2-(A^3)_j-T^1-(A^2)_i-T-A^1- \qquad II$$

wherein:

$R^3$ is selected from H, alkyl, alkoxy, alkylthio, fluoro-alkyl & -alkoxy, alkoxyalkyl, halogeno, alkyl-carbonyl & -carbonyloxy and alkoxy-carbonyl & -carbonyloxy;

T, $T^1$ & $T^2$ are independently selected from a single covalent bond, or a group selected from -CO.O-, -O.CO, -CO.S-, CS.O-, $-CH=CH-$, $-CH_2O-$, $-OCH_2-$, $-\underset{\substack{\downarrow\\ O}}{N}=N-$, $-N=\underset{\substack{\downarrow\\ O}}{N}-$, $-N=N-$, $-N=CH-$, $-CH=N-$, $-CH_2CHE-$, $-CHECH_2-$, and $-C\equiv C-$;

E is selected from H, cyano, halogen, $CF_3$ and $CH_3$;

$A^1$, $A^2$, $A^3$ & $A^4$ are independently selected from:

optionally substituted 1,4-phenylene optionally with one to four cyclic carbon atoms replaced by N; optionally substituted 1,4-cyclohexylene optionally with one or two cyclic carbon atoms replaced by O, S or N; 1,4-bicyclo(2,2,2)octane; 2,6-naphthylene or 1,4-naphthylene; and

i, j & k independently represent O or 1.

Where $R^3$ is other than H it preferably contains from 4 to 12 carbon atoms and more preferably at least 6 carbon atoms. It is preferred that alkyl chains are unbranched and especially preferred groups repre-

sented by $R^3$ are $C_{6-12}$-n-alkyl and $C_{6-12}$-n-alkoxy.

It is preferred that each of $T^1$ to $T^3$ is independently selected from -$CH_2$.$CH_2$-, -O.$CH_2$-, -$CH_2$.O-, -O.CO- and more especially from -CO.O-, -CO.S- and a single covalent bond.

It is preferred that E is H but where E is halogen this is preferably selected from F, Cl and Br.

Where any one of $A^1$ to $A^4$ represents a substituted 1,4-phenylene group, it may contain up to four substituents selected from $CH_3$, $OCH_3$, $CF_3$, CN, $NO_2$, F, Cl, Br, OH and $COCH_3$ in the 2, 3, 5 and/or 6 positions in order to modify the physical properties of the molecule, e.g. melting point. It is however, preferred that the group be unsubstituted.

Where any one of $A^1$ to $A^4$ represents phen-1,4-ylene having one or two cyclic carbon atoms replaced by nitrogen, it is preferably pyridinylene or 1,3-pyrimidinylene, especially pyridin-2,5-ylene or 1,3-pyrimidin-2,5-ylene.

Where any one of $A^1$ to $A^4$ represents cyclohex-1,4-ylene optionally having one or two cyclic carbon atoms replaced by O, S or N it is desirably in the trans configuration and preferably trans-1,3-dioxylene, especially trans-1,3-diox-2,5-ylene, trans-piperidin-1,4-ylene or trans-piperazin-1,4-ylene.

Where any one of $A^1$ to $A^4$ represents 1,4-cyclohexylene having one or more substituents, the substituents are preferably in the 1 and/or 4 positions, such as 1-cyanocyclohex-1,4-ylene.

It is preferred that the sum of i, j and k is 1, 2 or 3 and further preferred that two of i, j and k are 1.

A preferred compound according to the present invention conforms to the formula:

$$C_pH_{2p+1}-O-A^3-A^2-T-A^1-\underset{\substack{|\\ CN}}{\overset{\substack{R^1\\ |}}{C^*}}-R^2 \qquad III$$

wherein:

p is an integer from 6 to 12, especially from 7 to 12;

$R^1$ is selected from $C_{1-4}$ -alkyl and halogen and $R^2$ is selected from alkyl, alkoxy, alkylthio, halo-alkyl & -alkoxy, alkoyxalkyl, halogeno, alkyl-carbonyl & -carbonyloxy and alkoxy-carbonyl and -carbonyloxy, provided that $R^1$ and $R^2$ are different;

T is -CO.O- or -CO.S-; and

$A^1$, $A^2$ and $A^3$ are each independently selected from 1,4-phenylene optionally with one to four cyclic carbon atoms replaced by N; 1-4-cyclohexylene optionally with one or two cyclic carbon atoms replaced by O, S or N; 1,4-bicyclo(2,2,2)octane; and 1,6-naphthylene or 1,4-naphthylene.

It is preferred that $R^1$ is $C_{1-4}$-alkyl, especially $CH_3$, and that $R^2$ is alkyl, especially $C_{4-12}$-alkyl. Where $R^2$ is halo-alkoxy, it is preferably fluoro-alkyl or fluoro-alkoxy. It is especially preferred that $R^1$ is $CH_3$ and $R^2$ is $C_8H_{17}$. It is also preferred that not more than one of $R^1$ or $R^2$ is halogen, preferably F, Cl or Br.

Specific examples of compounds in accordance with Formula I and Formula III are:

2-cyano-2-(4-[4-(4-n-octyloxyphenyl)benzoyloxy]phenyl)decane; and 1-cyano-1-(4-[4-(4-n-octyloxyphenyl)-benzoyloxy]phenyl)nonane.

The compounds of Formula I can be derived by preparing separately the liquid crystal residue without the terminal cyclic group, $A^1$, and the chiral cyanoalkyl residue including the group $A^1$ as one of the substituents of the asymmetric carbon atom, thereafter coupling a single enantiomer of the chiral residue with the liquid crystal residue, in a coupling reaction determined by the link group, T, desired.

Thus a process for the preparation of a compound of Formula I in which the liquid crystal residues are represented by Formula II, may comprise reacting a compound of the formula:

$$R^3-(A^4)_k-T^2-(A^3)_j-T^1-(A^2)_i-CO.Q \qquad IV$$

such as

$$C_pH_{2p+1}-O-A^3-A^2-CO.Q \qquad V$$

wherein Q is OH or halogen,

with a single enantiomer of the formula:

$$HO-A^1-\overset{\overset{R^1}{|}}{\underset{\underset{CN}{|}}{C^*}}-R^2 \qquad VI$$

An example of such a process is the formation of (R)(or S)-2-cyano-2-(4-[4-(4-n-octyloxyphenyl)-benzoyloxy]phenyl)decane, described in detail hereinafter.

Because they contain an asymmetric carbon (C*) the compounds of Formula I, in optically pure form, exhibit a large spontaneous polarisation ($P_s$) when the molecules are arranged in a tilted smectic mesophase. Furthermore, some of these compounds, particularly those of Formula III, also exhibit a tilted smectic mesophase, especially a smectic C mesophase, at temperatures close to ambient.

A compound of Formula I which exhibits both these properties can be used as the active ingredient in a device, employing the ferroelectric effect for the modulation of electromagnetic radiation, which is operational at or close to ambient temperature. Compounds of Formula I which do not exhibit a tilted smectic mesophase over a convenient temperature range, can be used in a similar manner if they are formulated into a composition with other compounds with which they are compatible and which other compounds do exhibit the desired tilted smectic mesophase in a convenient temperature range. In such a composition the compound of Formula I provides the large spontaneous polarisation and the other compound orientates it in a tilted mesophase.

The invention is further illustrated by the following example in which all parts and percentages are by weight unless otherwise indicated.

Preparation of (R)(or S)-2-cyano-2-(4-[4-(4-n-octyloxyphenyl)benzoyloxy]phenyl decane

This preparation was carried out in a series of steps with the intermediate prepared in each step as specified in the subheadings.

(a) Ethyl (RS)-2-(4-methoxyphenyl),2-methyl decanoate

This was prepared by successively treating ethyl 4-methoxyphenylacetate (1.940 g, 10 mmol) with lithium diisopropylcyclohexylamide and iodooctane according to the published procedure (M W Rathke and A Lisdert, J.Am.Chem. Soc. 1971, 93, 2318) isolating the intermediate product and successively treating it with lithium diisopropylcyclohexylamide and iodomethane to give the product, after purification by flash column chromatography (eluant hexane:ether 10:1), as a colourless oil. Yield (2.30 g, 72%).

(b) (RS)-2-(4-methoxyphenyl), 2-methyl decanoyl chloride

A mixture of ethyl 2-(4-methoxyphenyl), 2-methyl decanoate (1.54 g, 4.8 mmol) and sodium hydroxide (0.768 g, 19.2 mmol) in ethanol (16 ml) and water (5 ml) were heated at reflux for 18 hours. The mixture was diluted with water (50 ml), acidified with concentrated hydrochloricacid and extracted into ether (3 x 50 ml). The combined organic extracts were dried (MgSO4) and evaporated in vacuo to yield the crude acid as a colourless oil (1.261 g, 90%).

A solution of the crude acid (1.379 g, 4.7 mmol) in thionyl chloride (10 ml) was stirred at room temperature for 1 hour. The excess thionyl chloride was evaporated in vacuo to yield the crude acid chloride as a yellow oil (1.53 g, 100%). The infra-red spectra of the starting ester, intermediate acid and the final product confirmed that the conversion to the acid chloride was complete.

(c) (R)-1-(1-napthyl)ethyl (R)-2-(4-methoxyphenyl), 2-ethyl decanamide and (d) (R) -1-(1-naphyl)ethyl (S)-2-(4-methoxyphenyl), 2-methyl decanamide

A solution of (R)-1-(1-napthyl)ethylamine (0.807 g, 4.7 mmol) and triethylamine (1.3 ml, 9.4 mmol) in dichloromethane (10 ml) was added to a solution of (RS)-2-(4-methoxyphenyl), 2-methyl decanoyl chloride (1.379 g, 4.7 mmol) in dichloromethane (10 ml). The mixture was stirred at room temperature for 1.5 hours, diluted with dichloromethane (30 ml), washed with water (10 ml), dried (MgSO4) and evaporated in vacuo to yield the crude amides. Flash column chromatography (eluant hexane:ether 6:1) not only purified the product but also separated the diastereomeric amides yielding 0.654 g (33%) of one diestereoisomer and 0.720 g (36%) of the other. The structure and diastereomeric purity of the products were confirmed by 'H nmr spectroscopy, the resonances which where assigned to the $CH_3CH$ protons and the $-OCH_3$ protons had clearly defined and different δ values.

(e) (R)(or S)-2-cyano, 2-(4-methoxyphenyl) decane

A solution containing only one of the diastereometrically pure amides (0.438 g, 1.0 mmol) in thionyl chloride (10 ml) was heated at reflux for 2 hours. The excess thionyl chloride was evaporated in vacuo and the crude product was purified by flash column chromatography (eluant hexane:ether 10:1). Yield (0.181 g, 71%).

(f) (R)(or S)-2-cyano, 2-(4-hydroxyphenyl) decane

A solution of (R)(or S)-2-cyano, 2-(4-methoxyphenyl)decane (0.282 g, 1.0 mmol) in dichloromethane (16 ml) was added to a solution of boron tribromide in dichloromethane (12 ml). The mixture was stirred at room temperature for 16 hours, cooled to 0 °C and water (10 ml) was added. The mixture was washed with saturated sodium bicarbonate solution, dried (MgSO4) and evaporated in vacuo. The crude material was purified by flash column chromatography (eluant hexane:ethyl acetate 6:1) to yield the phenol (0.257 g, 96%) as a colourless oil. The 'H n.m.r, ir, and mass spectra were consistent with the proposed structure.

(g) (R)(or S)-2-cyano-2-[4[4-(4-n-octyloxyphenyl)enzoyloxyl]-phenyl) decane

4-(4-n-octyloxyphenyl)-benzoic acid (0.326 g, 1.0 mmol) and thionyl chloride (4 ml) were heated at reflux for 15 mins, after which excess thionyl chloride was evaporated in vacuo. To a solution of this crude acid chloride in dichloromethane (5 ml) was added a solution of (R)(or S)-2-cyano, 2-(4-hydroxyphenyl)-decane (0.257 g, 0.99 mmol) and triethylamine (0.28 ml, 2.0 mmol) in dichloromethane (5 ml). The mixture was stirred for 1 hour at room temperature, diluted with dichloromethane (20 ml), washed with water (10 ml), dried (MgSO4), and evaporated in vacuo. The crude product was purified by flash column chromatography (eluant hexane:ethyl acetate 15:1) to yield the final product as a white solid. The structure of the product was confirmed by 'H nmr, ir, and mass spectra and by elemental analysis. On a rising temperature scale the compound forms a smectic C mesophase (Sc*) from its melting point (56 °C) through to 66 °C when it becomes isotropic. On a falling temperature scale the compound forms a Sc* mesophase between 66 °C and 50 °C. The spontaneous polarisation is 520 nc $cm^{-2}$ @ 50 °C and the tilt angle (25 °) is constant over the Sc* range. The response time to 50 V step in a 7.5 mm sample cell is 65 us @ 56 °C.

## Claims

1. A liquid crystal device comprising means enclosing a liquid crystal material and means for subjecting the liquid crystal material to an electric or magnetic field, the liquid crystal material comprising or containing a compound which has spontaneous polarisation properties and is of the formula:

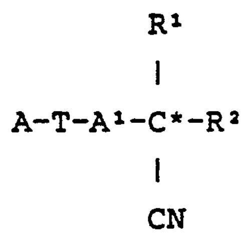

I

wherein : A-T-$A^1$ is the residue of a liquid crystal material or a liquid crystal compatible material, having a terminal cyclic structure, $A^1$, connected to the asymmetric carbon atom, $C^*$, by a single covalent bond;
$A^1$ is selected from: optionally substituted 1,4-phenylene optionally with one to four cyclic carbon atoms replaced by N; optionally substituted 1,4-cyclohexylene optionally with one or two cyclic carbon atoms replaced by O, S or N; 1,4-bicyclo (2,2,2) octane; 2,6-naphthylene or 1,4-naphthylene;
T is selected from a single covalent bond, or a group selected from -CO.O-, -O.CO, -CO.S-, CS.O-, -CH=CH-, $-CH_2O-$, $-OCH_2-$,$-\underset{\overset{\downarrow}{O}}{N}=N-$, $-N=\underset{\overset{\downarrow}{O}}{N}-$,
-N=N-, -N=CH-, -CH=N-, $-CH_2CHE-$, $-CHECH_2-$, and -C≡C-;
E is selected from H, cyano, halogen, $CF_3$ and $CH_3$;
$R^1$ is selected from $C_{1-4}$-alkyl and halogen;
$R^2$ is selected from alkyl, alkoxy, alkylthio,halo-alkyl & -alkoxy, alkoxyalkyl, halogeno, alkyl-carbonyl & -carbonyloxy and alkoxy-carbonyl & -carbonyloxy;
and A is the remainder of the liquid crystal residue or liquid crystal compatible material;
provided that $R^1$ and $R^2$ are different.

2. A device as claimed in Claim 1 in which said compound is one which exhibits a tilted smectic mesophase.

3. A device as claimed in Claim 1 in which the liquid crystal material comprises a composition containing a first compound which exhibits a titled smectic mesophase over a predetermined temperature range and a second compound of Formula I which exhibits spontaneous polarisation whereby the second compound provides the liquid crystal composition with a spontaneous polarisation characteristic while the first compound orientates the molecules of the second compound in a titled smectic mesophase.

4. A device as claimed in Claim 1 in which said compound of formula I conforms to the formula:

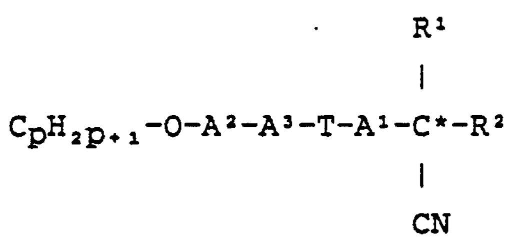

III

wherein: p is an integer from 6 to 12, especially from 7 to 12;
$R^1$ is selected from $C_{1-4}$-alkyl and halogen and $R^2$ is selected from alkyl, alkoxy, alkylthio, halo-alkyl & -alkoxy, alkoyxalkyl, halogeno, alkyl-carbonyl & -carbonyloxy and alkoxy-carbonyl & -carbonyloxy; provided that $R^1$ and $R^2$ are different;
T is -CO.O- or -CO.S-; and
$A^1$, $A^2$ & $A^3$ are each independently selected from 1,4-phenylene optionally with one to four cyclic carbon atoms replaced by N; 1-4-cyclohexylene optionally with one or two cyclic carbon atoms replaced by O, S or N; 1,4-bicyclo(2,2,2)octane; and 1,6-naphthylene or 1,4-naphthylene.

5. A device as claimed in Claim 4 in which said compound of formula 1 comprises 2-cyano-2-(4-[4-(4-n-octyloxphenyl)benzoyloxy]phenyl)decane.

6. A device as claimed in Claim 4 which said compound of formula I comprises 1-cyano-1-(4-[4-(4-n-octyloxyphenyl)benzolyloxy]phenyl)nonane.

7. A device as claimed in Claim 1 in which $A^1$ comprises optionally substituted 1,4-phenylene optionally with 1 to 4 carbon atoms replaced by N, $R^1$ is $C_{1-4}$- alkyl and $R^2$ is $C_4$-$C_{12}$-alkyl.

8. A compound of the formula:

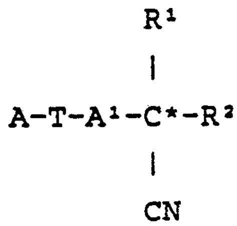

I

wherein : A-T-$A^1$ is the residue of a liquid crystal material or a liquid crystal compatible material, having a terminal cyclic structure, $A^1$, connected to the asymmetric carbon atom, C*, by a single covalent bond;
$A^1$ is selected from: optionally substituted 1,4-phenylene optionally with one to four cyclic carbon atoms replaced by N; optionally substituted phenylene optionally with one to four cyclic carbon atoms replaced by N; optionally substituted 1,4-cyclohexylene optionally with one or two cyclic carbon atoms replaced by O, S or N; 1,4-bicyclo (2,2,2) octane; 2,6-naphthylene or 1,4-naphthylene;
T is selected from a single covalent bond, or a group selected from -CO.O-, -O.CO, -CO.S-, CS.O-, -CH = CH-, $-CH_2O-$, $-OCH_2-$, -N(→O)=N-, -N(→O)=N-, -N = N-, -N = CH-, -CH = N-, $-CH_2CHE-$, $-CHECH_2-$, and -C≡C-;
E is selected from H, cyano, halogen, $CF_3$ and $CH_3$;
$R^1$ is selected from $C_{1-4}$-alkyl and halogen; and
$R^2$ is selected from alkyl, alkoxy, alkylthio, halo-alkyl & -alkoxy, alkoxyalkyl, halogeno, alkyl-carbonyl & -carbonyloxy and alkoxy-carbonyl & -carbonyloxy;
provided that $R^1$ and $R^2$ are different.

9. A compound as claimed in Claim 8 in which $A^1$ is an unsubstituted 1,4-phenylene group or a substituted 1,4-phenylene containing up to four substituents selected from $CH_3$, $OCH_3$, $CF_3$, CN, $NO_2$, F, Cl, Br, OH and $COCH_3$ in the 2, 3, 5 and/or 6 positions.

10. A compound as claimed in Claim 9 in which $A^1$ is a phen-1,4-ylene having one or two cyclic carbon atoms replaced by nitrogen.

11. A compound as claimed in Claim 8 in which $A^1$ is a cyclohex-1,4-ylene optionally having one or two cyclic carbon atoms replaced by O,S or N and is in the trans-configuration.

12. A compound as claimed in Claim 8 in which $A^1$ is 1,4-cyclohexylene having one or more substituents in the 1 and/or 4 positions.

13. A compound as claimed in Claim 8 in which $R^1$ is $C_{1-4}$-alkyl and $R^2$ is $C_4$-$C_{12}$ alkyl.

14. A compound as claimed in Claim 8 comprising 2-cyano -2-(4-[4-(4-n-octyloxyphenyl)benzoyloxy]-phenyl)decane.

15. A compound as claimed in in Claim 8 comprising 1-cyano-1-(4-[4-(4-n-octyloxyphenyl)benzoyloxy]-phenyl)nonane.

16. A process for the preparation of a compound as claimed in Claim 8 comprising reacting a compound of the formula:

$R^3$-$(A^4)_k$-$T^2$-$(A^3)_j$-$T^1$-$(A^2)_i$-CO.Q

with a single enantiomer of the formula:

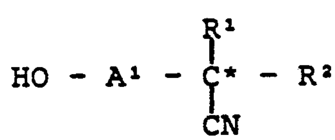

wherein:
Q is OH or halogen;
$R^1$ is selected from $C_{1-4}$ -alkyl or halogen;
$R^2$ is selected from alkyl, alkoxyl, alkylthio, halo-alkyl and -alkoxyl, alkoxyalkyl, halogeno, alkyl-carbonyl and -carbonyloxy and alkoxy -carbonyl and -carbonyloxy;
$R^3$ is selected from H, alkyl, alkoxy, alkylthio, fluoro-alkyl & -alkoxy, alkoxyalkyl, halogeno, alkyl-carbonyl & -carbonyloxy and alkoxy-carbonyl & -carbonyloxy;

T, $T^1$ & $T^2$ are independently selected from a single covalent bond, or a group selected from -CO.O-, -O.CO, -CO.S-, CS.O-, -CH=CH-, $-CH_2O-$, $-OCH_2-$, $-\underset{\substack{\downarrow\\O}}{N}=N-$, $-N=\underset{\substack{\downarrow\\O}}{N}-$, -N=N-, -N=CH-, -CH=N-, $-CH_2CHE-$, $-CHECH_2-$, and -C≡C-;

E is selected from H, cyano, halogen, CF3 and $CH_3$;

$A^1$, $A^2$, $A^3$ & $A^4$ are independently selected from: optionally substituted 1,4-phenylene optionally with one to four cyclic carbon atoms replaced by N; optionally substituted 1,4-cyclohexylene optionally with one or two cyclic carbon atoms replaced by 0, S or N; 1,4-bicyclo(2,2,2)octane; 2,6-naphthylene or 1,4-naphthylene; and

i, j & k independently represent O or 1.